# EUROPÄISCHE PATENTSCHRIFT

(11) **EP 3 772 345 B1**
(45) Veröffentlichungstag und Bekanntmachung des Hinweises auf die Patenterteilung: **22.12.2021**
(21) Anmeldenummer: 20176747.2
(22) Anmeldetag: 27.05.2020
(51) Int. Cl.: A61B 17/34, A61B 90/50

(54) **VORRICHTUNG ZUR SIMULTANEN FIXIERUNG VON MEDIZINISCHEN INSTRUMENTEN UND ENTSPRECHENDES SYSTEM**
DEVICE FOR THE SIMULTANEOUS FIXING OF MEDICAL INSTRUMENTS AND CORRESPONDING SYSTEM
DISPOSITIF DE FIXATION SIMULTANÉE DES INSTRUMENTS MÉDICAUX ET SYSTÈME CORRESPONDANT

(30) Priorität: 07.08.2019 DE 102019121360
(43) Veröffentlichungstag der Anmeldung: 10.02.2021
(73) Patentinhaber: Karl Storz SE & Co. KG, 78532 Tuttlingen (DE)
(72) Erfinder: Huber, Florian, 78532 Tuttlingen (DE); Göbel, Werner, 78532 Tuttlingen (DE); Glöggler, Bernhard, 78532 Tuttlingen (DE)
(74) Vertreter: Witte, Weller & Partner Patentanwälte mbB

(56) Entgegenhaltungen:
- WO-A1-2016/144180
- WO-A2-01/54560
- US-A1- 2012 296 281

## Beschreibung

Die vorliegende Erfindung betrifft eine Vorrichtung zur simultanen Fixierung von medizinischen Instrumenten sowie ein System mit einer solchen Vorrichtung.

Die Positionierung von medizinischen Instrumenten erfolgt in der klinischen Routine nach wie vor per Hand oder mit speziellen Haltearmsystemen. Dabei ist insbesondere die Positionierung eines in einem Trokar eingeführten Instruments von Interesse. Es sei dabei lediglich beispielhaft auf die Veröffentlichungen US 2012/0296281 A1 und WO 01/54560A2 verwiesen.

Zu den Anforderungen bei der Positionierung kann zum einen eine schnelle Möglichkeit der Positionierung in axialer Richtung, also entlang einer Koppellängsachse, insbesondere der Endoskopachse, zählen. Ferner ist oftmals eine Rotationsmöglichkeit um eine Einstichstelle, insbesondere einem Trokareinstich, gewünscht. Der Schwenkpunkt bzw. Pivotpunkt wird dabei bevorzugt in unmittelbarer Nähe der Einstichstelle gewählt, um bei einer Verlagerung eine mechanische Belastung, insbesondere eine Zerrung, an der Einstichstelle zu vermeiden.

WO 2016/144180 A1 zeigt einen maßgeschneiderten Trokar mit einem komprimierbaren Kugelgelenk. An dem Kugelgelenk ist ein Überwurfband angebracht, welches beim Zusammenziehen des Bandes eine Kugel des Kugelgelenks komprimiert und somit eine Fixierung eines in den Trokar eingeführten Instruments erzielt. Es verbleibt jedoch u.a. der Wunsch, dass die für das Halten verwendete Vorrichtung flexibler bezüglich verschiedener Instrumente ist, insbesondere im Hinblick auf einen Durchmesser der Instrumente.

Bei Lösungen, die kein Haltearmsystem benötigen, kann eine manuelle Positionierung des Endoskops bzw. Instruments schnell und der Situation entsprechend erfolgen, erfordert aber eine weitere Person im sterilen Bereich in einem Operationssaal zum Halten des Endoskops bzw. Instruments. Üblicherweise wird das Endoskop dabei gehalten und entsprechend geführt, während der Operateur mit weiteren Instrumenten (eingeführt durch weitere Trokare) unter dem Sichtfeld des Endoskops seine Eingriffe durchführt.

Haltearmsysteme andererseits erlauben eine Fixierung des Endoskops oder des Instruments, wobei das Lösen des Haltearms ggf. auch vom Operateur selbst vorgenommen werden kann. Dies erfordert jedoch in der Regel wiederum einen Eingriff in den Arbeitsablauf des Operateurs. Auch muss dabei üblicherweise ein komplettes Lösen des Haltearmmechanismus erfolgen, so dass eine Neupositionierung meist einer relativ zeitaufwändigen, kompletten Neupositionierung des Haltearms gleichkommt. Für diese Neupositionierung werden in der Regel beide Hände des Operateurs oder des Assistenten benötigt, sodass sie ihre aktuellen Tätigkeiten unterbrechen müssen.

Die Erfinder haben daher die Notwendigkeit eines einfach zu lösenden Haltesystems für in den Trokar eingeführte Endoskope oder Instrumente erkannt, welches die Möglichkeit eines Verschiebens des Endoskops oder des Instruments entlang einer Längsmittelachse, der z-Achse, sowie eine Verlagerung bzw. Rotation um einen Schwenkpunkt bzw. Pivotpunkt nahe dem Trokareinstich ermöglicht.

Dabei soll das Haltesystem grundsätzlich unabhängig vom verwendeten Trokar und Endoskop oder Instrument sein, z.B. hinsichtlich verschiedener Durchmesser oder Längen, um für eine Vielzahl möglicher Kombinationen, insbesondere von Trokar und Endoskop einsetzbar zu sein.

Es ist eine Aufgabe der vorliegenden Erfindung, eine Vorrichtung zur simultanen Fixierung von medizinischen Instrumenten aufzuzeigen, die eine Halterung von Instrumenten mit verschiedenen Durchmessern ermöglicht und mit einer Hand bedient werden kann. Ferner soll ein entsprechendes System mit zwei medizinischen Instrumenten aufgezeigt werden.

Gemäß einem ersten Aspekt wird die Aufgabe gelöst durch eine Vorrichtung zur simultanen Fixierung von medizinischen Instrumenten, die Vorrichtung mit einem Halteabschnitt, der zwei Halteelemente aufweist, einem Koppelelement, und einem Schubelement, wobei das Koppelelement dafür ausgebildet ist, dass der Halteabschnitt um eine Schwenkachse, insbesondere in einer Schwenkebene, verschwenkt werden kann, und das Schubelement dafür ausgebildet ist, in einer ersten Position keine Kraft oder eine erste Kraft auf den Halteabschnitt auszuüben, so dass der Halteabschnitt verschwenkt werden kann, und in einer zweiten Position eine zweite Kraft, die größer ist als die erste Kraft, auf den Halteabschnitt auszuüben, so dass der bzgl. einer Rotation um die Schwenkachse fixiert ist, wobei der Halteabschnitt einen durchgängigen Hohlraum mit einer Längsmittelachse aufweist, der für die Aufnahme eines ersten medizinischen Instruments entlang der Längsmittelachse ausgebildet ist, wobei jedes der Halteelemente ein Basiselement und ein Armelement aufweist, wobei jedes Armelement in einem ersten Armabschnitt ausgehend Basiselement sich von der Längsmittelachse entfernt, in einem zweiten Armabschnitt sich der Längsmittelachse annähert und mit einer Auflagefläche endet, wodurch ein Freiraum zwischen den Armelementen gebildet ist, der für die Aufnahme eines zweiten medizinischen Instruments entlang der Längsmittelachse ausgebildet ist.

Eine solche Vorrichtung ermöglicht es dem Operateur, auf einfache Weise eine Neupositionierung des Halteabschnitts vorzunehmen und damit die Lage der medizinischen Instrumente zu verändern. Dafür kann das Schubelement zwischen zumindest den zwei genannten Positionen hin und her bewegt und/oder gedreht werden. In der ersten Position übt das Schubelement keine oder nur eine geringe Kraft auf den Halteabschnitt aus. Dadurch kann der Halteabschnitt relativ zum Koppelelement frei bewegt werden oder unter einem geringen Widerstand bewegt werden. Diese erste Position kann demnach dafür genutzt werden, den Halteabschnitt erstmals zu positionieren oder neu zu positionieren. Die Schwenkachse wird bevorzugt dadurch realisiert, dass der Halteabschnitt an zwei oder mehr als zwei Punkten verschwenkbar gelagert ist.

In der zweiten Position übt das Schubelement eine größere zweite Kraft auf den Halteabschnitt aus. Dadurch entsteht einerseits eine größere Reibung, insbesondere Haftreibung, zwischen dem Schubelement und dem Halteabschnitt. Andererseits wird der Halteabschnitt auch gegen sein Lager gedrückt, was auch an dieser Stelle die Reibung erhöht. Diese Steigerung der Reibung führt dazu, dass der Halteabschnitt zumindest in dem Freiheitsgrad um die Schwenkachse fixiert ist. Die zweite Position wird also dann verwendet, wenn der Halteabschnitt wie gewünscht positioniert ist und dessen Lage bzgl. des Freiheitsgrads um die Schwenkachse, also insbesondere in der Schwenkebene, nicht mehr verändert werden soll.

Ferner sei darauf hingewiesen, dass bei Verwendung eines Haltearms der Halteabschnitt auch um eine Längsachse des Haltearms rotieren kann, wenn sich das Schubelement in der ersten Position befindet. Zudem ist es möglich, statt eines Endoskops auch andere zweite medizinische Instrumente zu halten, wie zum Beispiel Zangen oder Scheren.

Dadurch ist die Aufgabe vollständig gelöst.

Bei einer bevorzugten Ausgestaltung ist es möglich, über eine Variation der Position des Schubelements auch zu bestimmen, ob eines, zwei oder mehrere der medizinischen Instrumente entlang der Längsmittelachse verlagert werden können. So können zum einen die Halteelemente derart ausgebildet sein, dass sie in der ersten Position eine Verlagerung des darin geführten ersten medizinischen Instruments erlauben, nicht hingegen jedoch in der zweiten oder einer anderen dritten Position. Dies folgt daraus, dass die zwei Halteelemente weniger Kraft auf das erste medizinische Instrument in dem Hohlraum ausüben als in der zweiten oder dritten Position. Dabei kann bei einer bevorzugten Ausgestaltung wieder Reibung, insbesondere Haftreibung, dafür ursächlich, dass bei einer größeren Kraft das erste medizinische Instrument fixiert wird. Der Halteabschnitt wird insbesondere in einer Schwenkebene verschwenkt, die senkrecht zur Schwenkachse steht. Der Halteabschnitt wird dann bei seiner Fixierung bzgl. einer Rotation um die Schwenkachse gleichfalls bzgl. einer Verlagerung in der Schwenkebene fixiert. Bei einer anderen bevorzugten Ausgestaltung, ist das erste Instrument alternativ oder zusätzlich formschlüssig gehalten.

Bei einer bevorzugten Ausgestaltung üben die Armelemente mit ihren Auflageflächen in der zweiten oder einer dritten Position eine Kraft auf das zweite medizinische Instrument aus, sei es direkt durch einen physikalischen Kontakt oder indirekt über ein Zwischenelement. Während in der ersten Position die durch die Armelemente ausgeübte Kraft noch gering ist und ein Verlagern des zweiten medizinischen Instruments entlang der Längsmittelachse erlaubt, werden in der zweiten oder der dritten Position die Armelemente so in Richtung der Längsmittelachse gegen das zweite medizinische Instrument gedrückt, dass die nun erhöhte Reibung, insbesondere Haftreibung, eine Verlagerung des zweiten medizinischen Instruments entlang der Längsmittelachse verhindert.

Bei einer bevorzugten Ausgestaltung ist eine dritte Kraft in der dritten Position größer als die zweite Kraft. Auf diese Weise ist es möglich, dass in der ersten Position des Schubelements sowohl die medizinischen Instrumente entlang der Längsmittelachse als auch der Halteabschnitt um die Schwenkachse verlagerbar sind. In der zweiten Position drückt das Schubelement gegen den Halteabschnitt und fixiert diesen bzgl. einer Rotation um die Schwenkachse, wobei aber immer noch eine Verlagerung der medizinischen Instrumente entlang der Längsmittelachse möglich ist. Bei der größten dritten Kraft werden die zwei Halteelemente so zusammengedrückt und damit gegen die medizinischen Instrumente gedrückt, dass nun sowohl die medizinischen Instrumente entlang der Längsmittelachse fixiert sind als auch der Halteabschnitt bzgl. einer Rotation um die Schwenkachse fixiert ist.

Bei einer anderen bevorzugten Ausgestaltung ist die dritte Kraft in der dritten Position größer als die erste Kraft aber kleiner als die zweite Kraft. Diese Ausgestaltung ermöglicht es, dass in der ersten Position sowohl die medizinischen Instrumente entlang der Längsmittelachse verlagerbar sind als auch der Halteabschnitt um die Schwenkachse verschwenkt werden kann. In der dritten Position mit der dritten Kraft drückt das Schubelement die zwei Halteelemente so zusammen und diese damit gegen die medizinischen Instrumente, dass diese entlang der Längsmittelachse fixiert sind. Schließlich wird in der zweiten Position zudem noch der Halteabschnitt bzgl. einer Rotation um die Schwenkachse fixiert.

Bei einer vorteilhaften Ausgestaltung kann zudem auch in einer vierten Position eine vierte Kraft aufgebracht werden, wobei in der ersten Position mit der ersten Kraft alle Elemente, d.h. Halteabschnitt, erstes medizinisches Instrument und zweites medizinisches Instrument, verlagerbar bzw. verschwenkbar sind, in einer dritten Position alle Elemente bis auf eins verlagerbar bzw. verschwenkbar sind, in der vierten Position alle Elemente bis auf eins fixiert sind und schließlich in der zweiten Position alle Elemente fixiert sind. Diese Möglichkeit der schrittweisen Fixierung kann dadurch erzielt werden, indem die entsprechenden Reibungskräfte durch entsprechende Ausgestaltung der Elemente, einschließlich Wahl der Materialien und der Oberflächen, beeinflusst werden. Dafür werden einerseits die Reibung zwischen dem Halteabschnitt und der Kombination von Koppelelement und Schubelement, die Reibung zwischen dem Halteabschnitt und dem ersten medizinischen Instrument sowie die Reibung zwischen den Auflageflächen bzw. einem an den Auflageflächen angeordneten Zwischenstück und dem zweiten medizinischen Instrument konstruktiv eingestellt.

Bei einer weiteren vorteilhaften Ausgestaltung verfügt das Koppelelement über einen Freiheitsgrad, so dass das Koppelelement um seine Längsachse, nachfolgend Koppellängsachse genannt, bzw. um eine Achse senkrecht zur Schwenkachse gedreht werden kann. Diese Drehung findet dann üblicherweise in Bezug auf einen Haltearm statt. Da der Halteabschnitt vom Koppelelement gehalten wird, kann der Halteabschnitt noch flexibler verlagert werden, nämlich einerseits um die Schwenkachse und andererseits in einer Rotationsebene, die bei einer Rotation um die Koppellängsachse entsteht.

Bei einer weiteren vorteilhaften Ausgestaltung kann die Verlagerung des Schubelements, sei es in eine der zuvor genannten Positionen oder eine fünfte Position, bewirken, dass das Koppelelement bezüglich seiner Rotation um seine Koppellängsachse fixiert wird. Wenn das Schubelement nämlich gegen den Halteabschnitt drückt, drückt der Halteabschnitt aufgrund seiner Lagerung am Koppelelement auch gegen das Koppelelement. Wird das Koppelelement aufgrund dieser Kraft beispielsweise gegen eine Fläche gedrückt, bevorzugt gegen eine konische Fläche, so bewirkt die Reibung zwischen dem Koppelelement und dieser Fläche, dass das Koppelelement relativ zu dieser Fläche fixiert ist und sich nicht mehr um seine Koppellängsachse drehen kann. Auf diese Weise kann eine sehr große Flexibilität bei der Positionierung der medizinischen Instrumente erzielt werden.

Bei einer bevorzugten Ausgestaltung ist es in der ersten Position möglich, den Halteabschnitt um die Schwenkachse zu verlagern bzw. zu rotieren, die medizinischen Instrumente entlang der Längsmittelachse zu verlagern und das Koppelelement um seine Koppellängsachse zu drehen. In der zweiten Position ist der Halteabschnitt bzgl. einer Rotation um die Schwenkachse fixiert, die medizinischen Instrumente sind entlang der Längsmittelachse fixiert und das Koppelelement ist hinsichtlich seiner Koppellängsachse fixiert.

Bei einer bevorzugten Ausgestaltung, ist das Schubelement dafür ausgebildet, ein erstes Halteelement der Halteelemente gegen ein zweites Halteelement der Halteelemente zu drücken, wodurch sich auch die Auflagefläche des ersten Halteelements und die Auflagefläche des zweiten Halteelements aufeinander zubewegen.

Diese Ausgestaltung ermöglicht es, die medizinischen Instrumente relativ zum Halteabschnitt zu fixieren. In Abhängigkeit von der Ausgestaltung der Halteabschnitte und der Auflageflächen kann zudem eingestellt werden, ob bei einer bestimmten vorgegebenen Kraft nur das erste medizinische Instrument oder nur das zweite medizinische Instrument fixiert ist oder ob beide medizinischen Instrumente im Wesentlichen gleichzeitig fixiert werden.

Bei einer weiteren bevorzugten Ausgestaltung, weist die Vorrichtung ferner ein Führungselement auf, welches eine Öffnung aufweist, die senkrecht zur Längsmittelachse angeordnet ist, und das Führungselement liegt an den Auflageflächen an, wenn die zweite Kraft auf den Halteabschnitt wirkt.

Diese Ausgestaltung ermöglicht es, dass die Vorrichtung besonders einfach auf zweite medizinische Instrumente mit verschiedenen Durchmessern eingestellt werden kann. Grundsätzlich ist es möglich, dass die Auflageflächen direkt an dem zweiten medizinischen Instrument anliegen und dieses halten und/oder fixieren. Wenn die Armabschnitte eine gewisse Flexibilität aufweisen, ist es sogar möglich, zweite medizinische Instrumente mit unterschiedlichen Durchmessern zu verwenden. Das Führungselement bietet hier weitergehende Möglichkeiten für eine Adaption auf eine große Bandbreite von unterschiedlichen Durchmessern des zweiten medizinischen Instruments. Dabei wird die Ausgestaltung des Führungselements derart gewählt, dass das Führungselement einerseits an den Auflageflächen anliegt und andererseits, zumindest wenn das Schubelement in der zweiten Position ist, auch am zweiten medizinischen Instrument anliegt. Eine von den Auflageflächen ausgeübte Kraft wird dann über das Führungselement auf das zweite medizinische Instrument übertragen. Das Führungselement kann auch dann vorteilhaft sein, wenn ein direkter Kontakt zwischen den Auflageflächen und dem zweiten medizinischen Instrument nicht erwünscht ist. Vorteilhafterweise wird das Führungselement an die Auflageflächen und/oder an die zweiten Armabschnitte angeclipst. Auf diese Weise kann das Führungselement sehr einfach ausgetauscht werden.

Bei einer weiteren bevorzugten Ausgestaltung, weist das Koppelelement zwei Vorsprünge auf, die parallel zueinander sind und zwischen denen das Schubelement geführt ist.

Diese Ausgestaltung ermöglicht ein gute Halterung des Halteabschnitts. Insbesondere kann dabei eine gute Führung des Halteabschnitts um die Schwenkachse gewährleistet werden.

Bei einer weiteren bevorzugten Ausgestaltung,
- weist das Koppelelement zwei Aussparungen auf, die einander zugewandt sind, und wobei der Halteabschnitt an zwei gegenüberliegenden Seiten jeweils einen Vorsprung aufweist, der in eine der Aussparungen eingreift, oder
- weist das Koppelelement zwei Vorsprünge auf, die einander zugewandt sind, und wobei der Halteabschnitt an zwei gegenüberliegenden Seiten jeweils eine Aussparung aufweist, in die einer der Vorsprünge eingreift.

Diese Ausgestaltung ermöglicht es auf einfache Weise, die Lagerung des Halteabschnitts am Koppelelement zu realisieren.

Bei einer weiteren bevorzugten Ausgestaltung, weisen die Aussparungen einen Schlitz auf, der so ausgebildet ist, dass der Halteabschnitt aus den Aussparungen senkrecht zur Schwenkachse herausgeschoben werden kann.

Diese Ausgestaltung ist vorteilhaft, da die Vorrichtung so einfach verlegt werden kann, insbesondere um sie einem Desinfektionsprozess und/oder einem Autoklavierprozess zuzuführen. Die Aussparung und der Schlitz sind dabei bevorzugt so angeordnet, dass der Halteabschnitt während des Betriebs in einem Bereich der Koppelelements gehalten wird, in dem die Aussparungen nicht in den Schlitz gelangen können und somit auch nicht aus dem Schlitz herausgeschoben werden können.

Bei einer weiteren bevorzugten Ausgestaltung, schneidet die Längsmittelachse die Schwenkachse.

Diese Ausgestaltung ist vorteilhaft, weil auf diese Weise ein Punkt erzeugt wird, nämlich der Schnittpunkt von Längsmittelachse und Schwenkachse, insbesondere ein Pivotpunkt, der sowohl bei einer Verlagerung des Halteabschnitts um die Schwenkachse als auch bei einer Drehung des Koppelelements um seine Koppellängsachse unverändert bleibt. Dies hat u.a. den Vorteil, dass, wenn dieser Schnittpunkt während einer Operation nahe der Stelle des Trokareinstichs liegt, eine Verlagerung des Halteabschnitts um die Schwenkachse und/oder ein Drehen des Koppelelements nur eine geringe Auswirkung bezüglich einer Verlagerung der Stelle des Trokareinstichs haben.

Bei einer weiteren bevorzugten Ausgestaltung, weist die Vorrichtung ferner einen Haltearm auf, von dem das Schubelement beim Ausüben der zweiten Kraft gehalten ist, wobei das Koppelelement einen Flansch aufweist, der mit einem Überwurfelement an dem Haltearm befestigt ist.

Diese Ausgestaltung ermöglicht es, das Koppelelement drehbar zum Haltearm anzuordnen und gleichzeitig das Schubelement zum Fixieren des Koppelelements hinsichtlich seiner Drehbewegung zu fixieren. Dabei ist das Überwurfelement an dem Halteabschnitt befestigt, und der Flansch des Koppelelements befindet sich unterhalb des Überwurfelements. Drückt das Schubelement nun gegen den Halteabschnitt, so drückt der Halteabschnitt auch gegen das Koppelelement. Dies führt dazu, dass der Flansch des Koppelelements gegen das Überwurfelement gedrückt wird. Die so entstehende Reibung, insbesondere Haftreibung, zwischen dem Flansch und dem Überwurfelement blockiert eine Drehung des Koppelelements um seine Koppellängsachse. Dabei ist es vorteilhaft, wenn das Überwurfelement mittels eines Gewindes oder eines Bajonettverschlusses an dem Haltearm angeordnet ist. Dies ermöglicht es, auf einfache Weise das Koppelelement vom Haltearm zu trennen und dann sowohl das Überwurfelement als auch das Koppelelement einem Desinfektionsprozess und/oder einem Autoklavierprozess zuzuführen. Insbesondere kann das Überwurfelement als Überwurfmutter ausgestaltet sein.

Bei einer weiteren bevorzugten Ausgestaltung, ist das Schubelement über ein Kugelgelenk mit dem Haltearm gekoppelt.

Diese Ausgestaltung ermöglicht es, dass das Schubelement zusammen mit dem Koppelelement um die Koppellängsachse gedreht werden kann.

Bei einer weiteren bevorzugten Ausgestaltung, weist ein erstes Halteelement der Halteelemente eine Ausnehmung auf und weist ein zweites Halteelement der Halteelemente einen Vorsprung auf, wobei der Vorsprung in die Ausnehmung eingreift, wenn die zweite Kraft auf den Halteabschnitt wirkt.

Diese Ausgestaltung ermöglicht es, dass nur eines der Halteelemente an dem Koppelelement gelagert ist, wohingegen das andere Halteelement durch das Zusammenspiel von Vorsprung und Ausnehmung gehalten ist. Da das Schubelement während der Betriebs an dem anderen Halteelement anliegt, lösen sich die Halteelemente nicht voneinander.

Bei einer weiteren bevorzugten Ausgestaltung, weist jedes Armelement einen dritten Armabschnitt auf, der zwischen dem ersten Armabschnitt und dem zweiten Armabschnitt angeordnet ist und sich zumindest ungefähr parallel zu der Längsmittelachse erstreckt.

Diese Ausgestaltung ermöglicht einen kompakten Aufbau, der aber dennoch einen guten Zugriff auf die medizinischen Instrumente, insbesondere auf das erste medizinische Instrument, ermöglicht.

Bei einer weiteren bevorzugten Ausgestaltung, stehen der erste Armabschnitt und der zweite Armabschnitt jeweils zumindest ungefähr senkrecht zum dritten Armabschnitt.

Diese Ausgestaltung ermöglicht einen kompakten Aufbau, der aber dennoch einen guten Zugriff auf die medizinischen Instrumente, insbesondere auf das erste medizinische Instrument, ermöglicht.

Bei einer weiteren bevorzugten Ausgestaltung, ist in den Halteabschnitt ein elastisches Halteelement eingesetzt, das dafür ausgebildet ist, ein erstes Instrument der medizinischen Instrumente entlang der Koppellängsachse aufzunehmen.

Diese Ausgestaltung ermöglicht es, dass auf einfache Weise erste Instrumente mit unterschiedlichen Durchmessern verwendet werden können. Wird ein solches elastisches Halteelement eingesetzt, so drücken die Halteabschnitte über das elastische Halteelement gegen das erste medizinische Instrument und fixieren es auf diese Weise entlang der Längsmittelachse. Ein solches elastisches Halteelement hat bevorzugt die Form eines Zylindermantels, insbesondere mit einem Flansch um ein Durchrutschen zu verhindern.

Bei einer weiteren vorteilhaften Ausgestaltung ist der Halteabschnitt dafür ausgebildet, einen Trokar zu führen, und die Auflageflächen sind dafür ausgebildet, ein in den Trokar eingeführtes Endoskop zu halten.

Da die Instrumente an verschiedenen Stellen gehalten werden, ist eine besonders gute individuelle Positionierung und Fixierung möglich. Diese Ausgestaltung kann insbesondere auf einen typischen Anwendungsfall ausgerichtet werden. Dafür liegt der Innendurchmesser des Hohlraums bevorzugt zwischen 5 und 15 Millimetern, besonders bevorzugt zwischen 7 und 13 Millimetern und insbesondere zwischen 8 und 12 Millimetern. Der Außendurchmesser des Endoskops ist zumindest geringfügig geringer als der Innendurchmesser des Trokars und ist bevorzugt kleiner als 10 Millimeter, besonders bevorzugt kleiner als 7 Millimeter und insbesondere kleiner als 5 Millimeter.

Gemäß einem weiteren Aspekt wird die Aufgabe gelöst durch ein System mit einer zuvor beschriebenen Vorrichtung, einem Trokar und einem Endoskop, wobei der Trokar von dem Halteabschnitt entlang der Längsmittelachse gehalten ist und das Endoskop in den Trokar eingeführt ist und durch eine von den Auflageflächen ausgeübte Kraft gehalten ist.

Einer der Aspekte der Erfindung liegt darin, dass an einen im Grunde beliebigen Trokar ein aus zwei Halteelementen bestehenden Klemmmechanismus angebracht wird. Die beiden Halteelemente sind relativ zum Koppelelement beweglich gelagert und lassen sich um die Schwenkachse rotieren. Dazu können die Halteelemente einen zylindrischen oder teilzylindrischen Vorsprung oder Ausnehmung aufweisen. Jedes der einzelnen Halteelemente verfügt wiederum um einen fingerähnlichen Fortsatz, das Armelement, welcher wiederum an das Instrument, insbesondere Endoskop, angreift (ggf. mit einem Zwischenstück oder Führungselement).

Bei einer mechanisch erzeugten Pressung der Halteelemente zueinander wird wiederum der erzeugte Druck über die Armelement an das Instrument übertragen, so dass dieses wiederum in der momentanen Position fixiert bleibt. Hierfür ist mindestens eines der beiden Halteelemente beweglich angebracht. Das Koppelelement selbst lässt sich um eine Achse senkrecht zur Schwenkachse der beiden Halteelemente rotieren. Dies führt dazu, dass die Halteelemente und damit auch der Trokar und das Instrument eine pivotierende Bewegung um den Schnittpunkt beider Achsen durchführen. Dieser Schnittpunkt liegt bei einigen Ausgestaltungen im Zentrum des Trokars.

Bei einer bevorzugten Ausführung kann die Reaktionskraft der Pressung der Halteelemente für die Fixierung des Koppelelements bezüglich einer ortsfesten Basis verwendet werden. Das System kann dabei mit unterschiedlichen Trokaren verwendet werden (ggf. mit einem elastischen Halteelement, z.B. eine Adapterhülse) und für unterschiedliche Instrumente verwendet werden (ggf. mit einem Führungselement oder mit Zwischenringen für unterschiedliche Durchmesser).

Die Armelemente zum Übertragen der Presskraft auf das Instrument, insbesondere Endoskop, können dabei bevorzugt abnehmbar ausgeführt sein oder aber an den Basiselementen gelagert sein, so dass die Armelemente nach außen weggeklappt werden können. Alternativ sind die Armelemente fest mit den Basiselementen verbunden. Hier können bevorzugt die Basiselemente mit den Armelementen wiederum an einem Zwischenadapter am Instrument gelagert sein.

Nachdem der Trokar gesetzt wurde, können die Halteelemente um diesen herum zusammengesetzt werden. Dazu können Elemente vorhanden sein, welche die Halteelemente nach dem Zusammenstecken zu einer selbst zusammenhaltenden Einheit werden lassen. Diese Elemente können z.B. aus Nuten, Dornen oder federnden Elementen bestehen.

Im nächsten Schritt kann das ein- oder auch mehrteilige Koppelelement, je nach gewünschter Ausführung, um die Halteelemente herum gelegt, geklappt oder aufgeschoben werden. In einem letzten Schritt wird dann bevorzugt ein Aktivator, bei dem es sich z.B. um einen pneumatischen Kolben, einen Piezoaktor oder einen Elektromotor handeln kann, mit dem Schubelement, ggf. auch mit dem Koppelelement, verbunden.

Eines der Ziele ist es, mittels eines geeigneten mechanischen Rotations- und Haltemechanismus dem Operateur die Möglichkeit zu geben, durch einfaches Lösen dieses Mechanismus ein in einen Trokar eingeführtes Instrument, insbesondere Endoskop, in axialer Richtung zu verschieben sowie eine Rotation um einen Pivotpunkt zu ermöglichen. Nach Schließen des Mechanismus soll das Instrument als auch der Trokar in der eingestellten Position fixiert sein. Das gesamte Haltesystem soll sich problemlos in den bekannten Operationsablauf einfügen und einfach auf- und abbaubar sein. Der Mechanismus kann dabei z.B. an einem herkömmlichen Haltearm fixiert sein. Das Öffnen und Schließen des Mechanismus soll bevorzugt einhändig und bevorzugt über eine geeignete Fernbedienung möglich sein, welche z.B. am Endoskop oder Instrument angebracht werden kann.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung näher dargestellt und werden in der nachfolgenden Beschreibung näher erläutert. Es zeigen:
- Fig. 1: eine Haltevorrichtung mit einem daran gekoppelten System;
- Fig. 2: eine Ausführungsform der Vorrichtung;
- Fig. 3: die Ausführungsform gemäß Fig. 2, wobei das Führungselement und das Koppelelement entfernt wurden;
- Fig. 4: ein erstes Halteelement der zwei Halteelemente aus Fig. 2;
- Fig. 5: ein zweites Halteelement der zwei Halteelemente aus Fig. 2;
- Fig. 6: das Schubelement und das Koppelelement aus Fig. 2 aus einer ersten Perspektive; und
- Fig. 7: das Schubelement, das Koppelelement und das Überwurfelement aus Fig. 2 aus einer zweiten Perspektive.

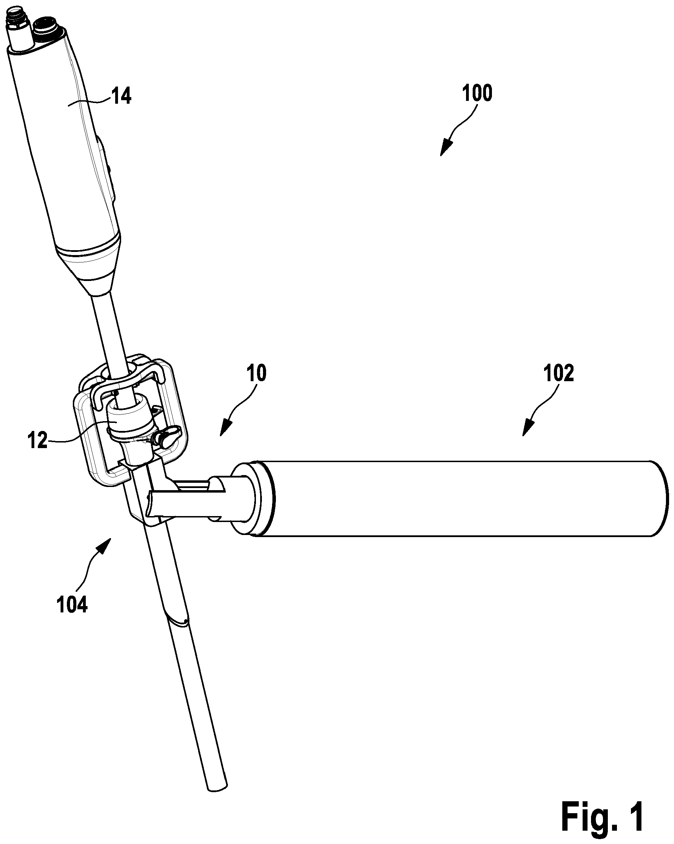

Fig. 1 zeigt einen Apparat 100, der einen Haltearm 102 und ein System 104 aufweist. Das System 104 weist eine Vorrichtung 10 auf, die ein erstes medizinisches Instrument 12, hier ein Trokar, und ein zweites medizinisches Instrument 14, hier ein Endoskop, fixiert. Die Vorrichtung 10 wird nun nachfolgend genauer erläutert.

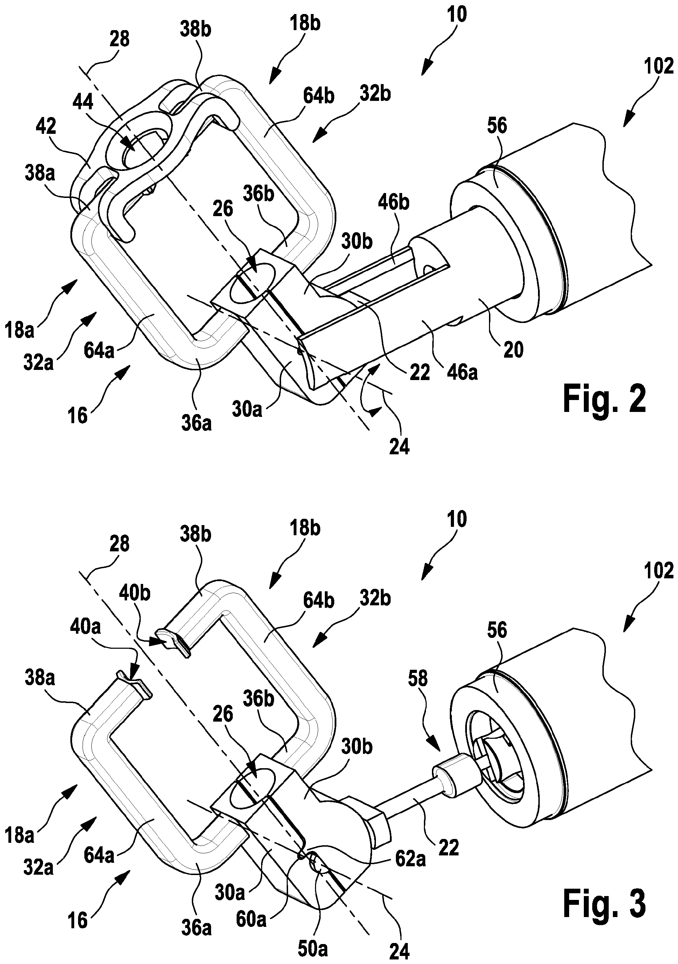

Fig. 2 zeigt die Vorrichtung 10 zur simultanen Fixierung von medizinischen Instrumenten 12, 14 aus Fig. 1. Die Vorrichtung 10 weist einen Halteabschnitt 16 auf, der zwei Halteelemente 18a, 18b aufweist, ein Koppelelement 20, und ein Schubelement 22 (siehe auch Fig. 3). Das Koppelelement 20 ist dafür ausgebildet, dass der Halteabschnitt 16 um eine Schwenkachse 24 in einer Schwenkebene verschwenkt werden kann, was mit dem Doppelpfeil symbolisch angedeutet ist. Das Schubelement 22 hat hier die Form eines Stempels.

Das Schubelement 22 ist dafür ausgebildet, in einer ersten Position keine Kraft oder eine erste Kraft auf den Halteabschnitt 16 auszuüben, so dass der Halteabschnitt 16 verschwenkt werden kann, und in einer zweiten Position eine zweite Kraft, die größer ist als die erste Kraft, auf den Halteabschnitt 16 auszuüben, so dass der Halteabschnitt 16 bzgl. einer Rotation um die Schwenkachse, also in der Schwenkebene, fixiert ist. Der Halteabschnitt 16 weist einen durchgängigen Hohlraum 26 mit einer Längsmittelachse 28 auf, der für die Aufnahme des ersten medizinischen Instruments 12 entlang der Längsmittelachse 28 ausgebildet ist.

Jedes der Halteelemente 18a, 18b weist ein entsprechendes Basiselement 30a, 30b und ein Armelement 32a, 32b auf, wobei jedes Armelement 32a, 32b in einem ersten Armabschnitt 36a, 36b ausgehend Basiselement 30a, 30b sich von der Längsmittelachse 28 entfernt, in einem zweiten Armabschnitt 38a, 38b sich der Längsmittelachse 28 annähert und mit einer Auflagefläche 40a, 40b endet, wodurch ein Freiraum zwischen den Armelementen 32a, 32b gebildet ist, der für die Aufnahme des zweiten medizinischen Instruments 14 entlang der Längsmittelachse 28 ausgebildet ist.

Das Schubelement 22 ist dafür ausgebildet, ein zweites Halteelement 18b der Halteelemente 18a, 18b gegen ein erstes Halteelement 18a der Halteelemente 18a, 18b zu drücken, wodurch sich auch die Auflagefläche 40a des ersten Halteelements 18a und die Auflagefläche 40b des zweiten Halteelements 18b aufeinander zubewegen.

Die Vorrichtung 10 weist ferner ein Führungselement 42 auf, welches eine Öffnung 44 aufweist, die senkrecht zur Längsmittelachse 28 angeordnet ist, wobei das Führungselement 42 an den Auflageflächen 40a, 40b anliegt, zumindest wenn die zweite Kraft auf den Halteabschnitt 16 wirkt.

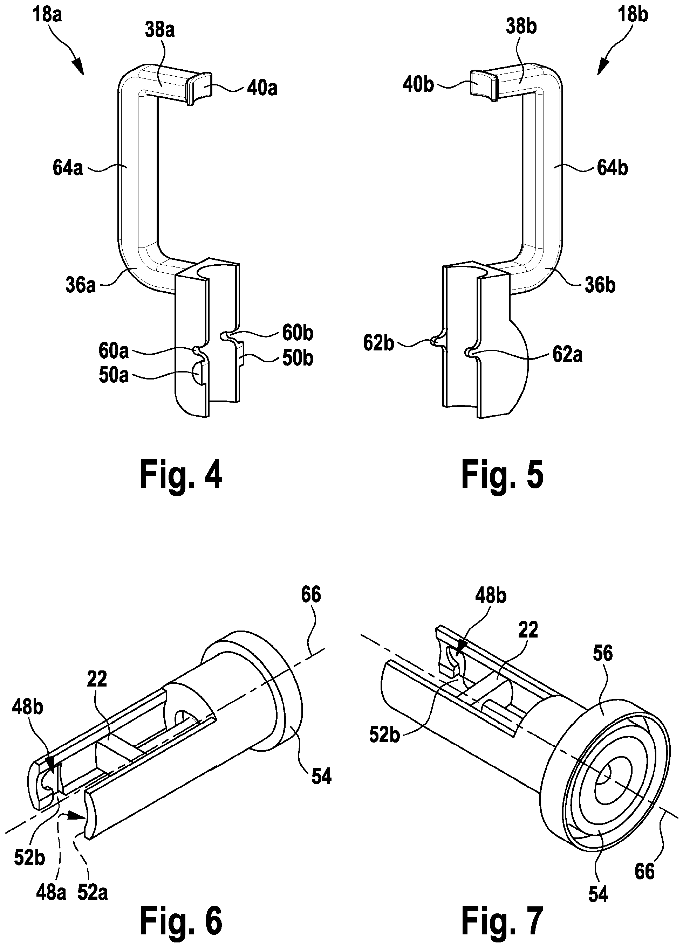

Das Koppelelement 20 weist zwei Vorsprünge 46a, 46b auf, die parallel zueinander sind und zwischen denen das Schubelement 22 geführt ist, hier in jeweils einer Vertiefung geführt ist. Das Koppelelement 20 weist außerdem zwei Aussparungen 48a, 48b (siehe Fig. 6) auf, die einander zugewandt sind, wobei der Halteabschnitt 16 an zwei gegenüberliegenden Seiten jeweils einen Vorsprung 50a, 50b (siehe Fig. 4) aufweist, der in eine der Aussparungen 48a, 48b eingreift.

Die Aussparungen 48a, 48b weisen jeweils einen Schlitz 52a, 52b auf, der so ausgebildet ist, dass der Halteabschnitt 16 aus den Aussparungen 48a, 48b senkrecht zur Schwenkachse 24 herausgeschoben werden kann. Bevorzugt weisen die Vorsprünge 50a, 50b dann eine Halbzylinderform auf. Es ist ferner nur erkennen, dass die Längsmittelachse 28 die Schwenkachse 24 schneidet.

Der Haltearm 102 hält bzw. stützt das Schubelement 22 beim Ausüben der zweiten Kraft. Das Koppelelement 20 weist einen Flansch 54 (siehe Fig. 6) auf, der mit einem Überwurfelement 56 (siehe Fig. 7) an dem Haltearm 102 befestigt ist.

In Fig. 3 ist erneut die Ausführungsform gemäß Fig. 2 gezeigt, wobei aber das Führungselement 42 das Koppelelement 20 nicht dargestellt sind. Es ist zu erkennen, dass das Schubelement 22 über ein Kugelgelenk 58 mit dem Haltearm 102 gekoppelt ist. Im Haltearm 102 befindet sich ein Aktor (nicht gezeigt), insbesondere ein Elektromotor, mit dem das Schubelement 22 zwischen zumindest der ersten Position und der zweiten Position hin und her bewegt werden kann.

Das erste Halteelement 18a der Halteelemente 18a, 18b weist zwei Ausnehmungen 60a, 60b auf und das zweite Halteelement 18b der Halteelemente 18a, 18b weist zwei Vorsprünge 62a, 62b auf, wobei der jeweilige Vorsprung 62a, 62b in die jeweilige Ausnehmung 60a, 60b eingreift, zumindest wenn die zweite Kraft auf den Halteabschnitt 16 wirkt.

Jedes Armelement 32a, 32b weist einen dritten Armabschnitt 64a, 64b auf, der zwischen dem jeweiligen ersten Armabschnitt 36a, 36b und dem jeweiligen zweiten Armabschnitt 38a, 38b angeordnet ist und sich insbesondere zumindest ungefähr parallel zu der Längsmittelachse 28 erstreckt. Dabei stehen insbesondere der jeweilige erste Armabschnitt 36a, 36b und der jeweilige zweite Armabschnitt 38a, 38b jeweils zumindest ungefähr senkrecht zum dritten Armabschnitt 64a, 64b.

Der Halteabschnitt 16 ist dafür ausgebildet, einen Trokar zu führen, und die Auflageflächen 40a, 40b sind dafür ausgebildet, ein in den Trokar eingeführtes Endoskop zu halten.

Fig. 4 zeigt das erste Halteelement 18a der zwei Halteelemente 18a, 18b aus Fig. 2. Die Vorsprünge 50a, 50b und die Ausnehmungen 60a, 60b sind hier gut zu erkennen.

Fig. 5 zeigt das zweite Halteelement 18b der zwei Halteelemente 18a, 18b aus Fig. 2. Die Vorsprünge 62a, 62b sind hier gut zu erkennen. Das zweite Halteelement 18b hat in seinem Abschnitt, der mit dem Schubelement 22 zusammenwirkt, eine Rundung, so dass der Halteabschnitt 16 bei seiner Verlagerung am Schubelement 22 entlanggleiten kann.

Fig. 6 zeigt das Schubelement 22 und das Koppelelement 20 aus Fig. 2 aus einer ersten Perspektive. Die Aussparung 48b mit dem Schlitz 52b ist gut zu erkennen. Die Aussparung 48a mit dem Schlitz 52a ist in dieser Perspektive verdeckt.

Fig. 7 zeigt das Schubelement 22, das Koppelelement 20 und das Überwurfelement 56 aus Fig. 2 aus einer zweiten Perspektive.

## Patentansprüche

1. Vorrichtung (10) zur simultanen Fixierung von medizinischen Instrumenten (12, 14), die Vorrichtung (10) mit einem Halteabschnitt (16), der zwei Halteelemente (18a, 18b) aufweist, einem Koppelelement (20), und einem Schubelement (22), wobei das Koppelelement (20) dafür ausgebildet ist, dass der Halteabschnitt (16) um eine Schwenkachse (24) verschwenkt werden kann, und das Schubelement (22) dafür ausgebildet ist, in einer ersten Position keine Kraft oder eine erste Kraft auf den Halteabschnitt (16) auszuüben, so dass der Halteabschnitt (16) verschwenkt werden kann, und in einer zweiten Position eine zweite Kraft, die größer ist als die erste Kraft, auf den Halteabschnitt (16) auszuüben, so dass der Halteabschnitt (16) bzgl. einer Rotation um die Schwenkachse fixiert ist, wobei der Halteabschnitt (16) einen durchgängigen Hohlraum (26) mit einer Längsmittelachse (28) aufweist, der für die Aufnahme eines ersten medizinischen Instruments (12) entlang der Längsmittelachse (28) ausgebildet ist, wobei jedes der Halteelemente (18a, 18b) ein Basiselement (30a, 30b) und ein Armelement (32a, 32b) aufweist, wobei jedes Armelement (32a, 32b) in einem ersten Armabschnitt (36a, 36b) ausgehend vom Basiselement (30a, 30b) sich von der Längsmittelachse (28) entfernt, in einem zweiten Armabschnitt (38a, 38b) sich der Längsmittelachse (28) annähert und mit einer Auflagefläche (40a, 40b) endet, wodurch ein Freiraum zwischen den Armelementen (32a, 32b) gebildet ist, der für die Aufnahme eines zweiten medizinischen Instruments (14) entlang der Längsmittelachse (28) ausgebildet ist.

2. Vorrichtung nach Anspruch 1, wobei das Schubelement (22) dafür ausgebildet ist, ein erstes Halteelement (18a) der Halteelemente (18a, 18b) gegen ein zweites Halteelement (18b) der Halteelemente (18a, 18b) zu drücken, wodurch sich auch die Auflagefläche (40a) des ersten Halteelements (18a) und die Auflagefläche (40b) des zweiten Halteelements (18b) aufeinander zubewegen.

3. Vorrichtung nach einem der vorhergehenden Ansprüche, wobei die Vorrichtung (10) ferner ein Führungselement (42) aufweist, welches eine Öffnung (44) aufweist, die senkrecht zur Längsmittelachse (28) angeordnet ist, und das Führungselement (42) an den Auflageflächen (40a, 40b) anliegt, wenn die zweite Kraft auf den Halteabschnitt (16) wirkt.

4. Vorrichtung nach einem der vorhergehenden Ansprüche, wobei das Koppelelement (20) zwei Vorsprünge (46a, 46b) aufweist, die parallel zueinander sind und zwischen denen das Schubelement (22) geführt ist.

5. Vorrichtung nach einem der vorhergehenden Ansprüche, wobei das Koppelelement (20) zwei Aussparungen (48a, 48b) aufweist, die einander zugewandt sind, und wobei der Halteabschnitt (16) an zwei gegenüberliegenden Seiten jeweils einen Vorsprung (50a, 50b) aufweist, der in eine der Aussparungen (48a, 48b) eingreift, oder wobei das Koppelelement (20) zwei Vorsprünge aufweist, die einander zugewandt sind, und wobei der Halteabschnitt (16) an zwei gegenüberliegenden Seiten jeweils eine Aussparung aufweist, in die einer der Vorsprünge eingreift.

6. Vorrichtung nach Anspruch 5, wobei die Aussparungen (48a, 48b) einen Schlitz (52a, 52b) aufweisen, der so ausgebildet ist, dass der Halteabschnitt (16) aus den Aussparungen (48a, 48b) senkrecht zur Schwenkachse (24) herausgeschoben werden kann.

7. Vorrichtung nach einem der vorhergehenden Ansprüche, wobei die Längsmittelachse (28) die Schwenkachse (24) schneidet.

8. Vorrichtung nach einem der vorhergehenden Ansprüche, ferner mit einem Haltearm (102), von dem das Schubelement (22) beim Ausüben der zweiten Kraft gehalten ist, wobei das Koppelelement (20) einen Flansch (54) aufweist, der mit einem Überwurfelement (56) an dem Haltearm (102) befestigt ist.

9. Vorrichtung nach einem der vorhergehenden Ansprüche, wobei das Schubelement (22) über ein Kugelgelenk (58) mit dem Haltearm (102) gekoppelt ist.

10. Vorrichtung nach einem der vorhergehenden Ansprüche, wobei ein erstes Halteelement (18a) der Halteelemente (18a, 18b) eine Ausnehmung (60a) aufweist und ein zweites Halteelement (18b) der Halteelemente (18a, 18b) einen Vorsprung (62a) aufweist, wobei der Vorsprung (62a) in die Ausnehmung (60a) eingreift, wenn die zweite Kraft auf den Halteabschnitt (16) wirkt.

11. Vorrichtung nach einem der vorhergehenden Ansprüche, wobei jedes Armelement (32a, 32b) einen dritten Armabschnitt (64a, 64b) aufweist, der zwischen dem ersten Armabschnitt (36a, 36b) und dem zweiten Armabschnitt (38a, 38b) angeordnet ist und sich insbesondere zumindest ungefähr parallel zu der Längsmittelachse (28) erstreckt.

12. Vorrichtung nach Anspruch 11, wobei der erste Armabschnitt (36a, 36b) und der zweite Armabschnitt (38a, 38b) jeweils zumindest ungefähr senkrecht zum dritten Armabschnitt (64a, 64b) stehen.

13. Vorrichtung nach einem der vorhergehenden Ansprüche, wobei in den Halteabschnitt (16) ein elastisches Halteelement eingesetzt ist, das dafür ausgebildet ist, ein erstes Instrument (12) der medizinischen Instrumente (12, 14) entlang der Längsmittelachse (28) aufzunehmen.

14. Vorrichtung nach einem der vorhergehenden Ansprüche, wobei der Halteabschnitt (16) dafür ausgebildet ist, einen Trokar zu führen, und die Auflageflächen (40a, 40b) dafür ausgebildet sind, ein in den Trokar eingeführtes Endoskop zu halten.

15. System (104) mit einer Vorrichtung (10) nach einem der vorhergehenden Ansprüche, einem Trokar und einem Endoskop, wobei der Trokar von dem Halteabschnitt (16) entlang der Längsmittelachse (28) gehalten ist und das Endoskop in den Trokar eingeführt ist und durch eine von den Auflageflächen (40a, 40b) ausgeübte Kraft gehalten ist.

## Claims

1. A device (10) for simultaneous fixation of medical instruments (12, 14), the device (10) comprising a holding portion (16) having two holding elements (18a, 18b), a coupling element (20), and a pushing element (22), wherein the coupling element (20) is configured so that the holding portion (16) can be pivoted about a pivot axis (24), and the pushing element (22) is configured, in a first position, not to exert a force or a first force on the holding portion (16) so that the holding portion (16) can be pivoted, and, in a second position, to exert a second force, which is greater than the first force, on the holding portion (16) so that the holding portion (16) is fixed regarding a rotation around the pivot axis, said holding portion (16) comprising a continuous cavity (26) having a longitudinal center axis (28) and being configured to receive a first medical instrument (12) along said longitudinal center axis (28), each of said holding members (18a, 18b) comprising a base member (30a, 30b) and an arm member (32a, 32b), each arm member (32a, 32b) having a first arm section (36a, 36b) starting from the base element (30a, 30b) extending away from the longitudinal center axis (28), a second arm section (38a, 38b) approaching the longitudinal center axis (28) and ending with a support surface (40a, 40b), whereby a free space is formed between the arm elements (32a, 32b), which is configured for receiving a second medical instrument (14) along the longitudinal center axis (28).

2. The device according to claim 1, wherein the pushing element (22) is configured to push a first holding element (18a) of the holding elements (18a, 18b) against a second holding element (18b) of the holding elements (18a, 18b), whereby the bearing surface (40a) of the first holding element (18a) and the bearing surface (40b) of the second holding element (18b) also move towards each other.

3. The device according to one of the preceding claims, wherein the device (10) further comprises a guide element (42) having an opening (44) arranged perpendicular to the longitudinal center axis (28), and the guide element (42) abuts against the bearing surfaces (40a, 40b) when the second force acts on the holding portion (16).

4. The device according to one of the preceding claims, wherein the coupling element (20) has two projections (46a, 46b) which are parallel to each other and between which the pushing element (22) is guided.

5. The device according to one of the preceding claims, wherein the coupling element (20) has two recesses (48a, 48b) facing each other, and wherein the holding portion (16) has on two opposite sides a respective projection (50a, 50b) which engages in one of the recesses (48a, 48b), or wherein the coupling element (20) has two projections facing each other, and wherein the holding portion (16) has on two opposite sides a respective recess in which one of the projections engages.

6. The device according to claim 5, wherein the recesses (48a, 48b) have a slot (52a, 52b) which is formed in such a way that the holding portion (16) can be pushed out of the recesses (48a, 48b) perpendicularly to the pivot axis (24).

7. The device according to one of the previous claims, where the longitudinal center axis (28) intersects the pivot axis (24).

8. The device according to one of the preceding claims, further comprising a holding arm (102) by which the pushing element (22) is held when the second force is exerted, wherein the coupling element (20) has a flange (54) which is attached to the holding arm (102) by a union element (56).

9. The device according to one of the preceding claims, wherein the pushing element (22) is coupled to the holding arm (102) via a ball joint (58).

10. The device according to one of the preceding claims, wherein a first holding member (18a) of the holding members (18a, 18b) has a recess (60a) and a second holding member (18b) of the holding members (18a, 18b) has a projection (62a), the projection (62a) engaging the recess (60a) when the second force acts on the holding portion (16).

11. The device according to one of the preceding claims, each arm element (32a, 32b) having a third arm portion (64a, 64b) which is arranged between the first arm portion (36a, 36b) and the second arm portion (38a, 38b) and in particular extends at least approximately parallel to the longitudinal center axis (28).

12. The device according to claim 11, wherein the first arm portion (36a, 36b) and the second arm portion (38a, 38b) are each at least approximately perpendicular to the third arm portion (64a, 64b).

13. The device according to one of the preceding claims, wherein an elastic holding element is inserted into the holding portion (16) which is configured to receive a first instrument (12) of the medical instruments (12, 14) along the longitudinal center axis (28).

14. The device according to any of the foregoing claims, wherein the holding portion (16) is configured to guide a trocar and the bearing surfaces (40a, 40b) are configured to hold an endoscope inserted into the trocar.

15. A system (104) comprising a device (10) according to any of the foregoing, a trocar and an endoscope, wherein the trocar is held by the holding portion (16) along the longitudinal center axis (28) and the endoscope is inserted into the trocar and held by a force exerted by the bearing surfaces (40a, 40b).

## Revendications

1. Dispositif (10) pour la fixation simultanée d'instruments médicaux (12, 14), le dispositif (10) étant pourvu d'une section de retenue (16), qui comprend deux éléments de retenue (18a, 18b), d'un élément de couplage (20) et d'un élément de poussée (22), l'élément de couplage (20) étant configuré de telle sorte que la section de retenue (16) peut être amenée à pivoter autour d'un axe de pivotement (24), et l'élément de poussée (22) étant configuré de manière à, dans une première position, n'exercer aucune force ou exercer une première force sur la section de retenue (16), de telle sorte que la section de retenue (16) peut être amenée à pivoter et, dans une deuxième position, exercer une deuxième force, qui est supérieure à la première force, sur la section de retenue (16), de telle sorte que la section de retenue (16) est fixée au regard d'une rotation autour de l'axe de pivotement, la section de retenue (16) comprenant une cavité traversante (26) ayant un axe médian longitudinal (28), qui est configurée pour la réception d'un premier instrument médical (12) le long de l'axe médian longitudinal (28), chacun des éléments de retenue (18a, 18b) comprenant un élément de base (30a, 30b) et un élément bras (32a, 32b), chaque élément bras (32a, 32b) s'éloignant de l'axe médian longitudinal (28) dans une première section de bras (36a, 36b) en partant de l'élément de base (30a, 30b), se rapprochant de l'axe médian longitudinal (28) dans une deuxième section de bras (38a, 38b) et se terminant avec une surface d'appui (40a, 40b), un espace libre entre les éléments bras (32a, 32b) étant ainsi formé, qui est configuré pour la réception d'un deuxième instrument médical (14) le long de l'axe médian longitudinal (28).

2. Dispositif selon la revendication 1, dans lequel l'élément de poussée (22) est configuré de manière à presser un premier élément de retenue (18a) parmi les éléments de retenue (18a, 18b) contre une deuxième élément de retenue (18b) parmi les éléments de retenue (18a, 18b), la surface d'appui (40a) du premier élément de retenue (18a) et la surface d'appui (40b) du deuxième élément de retenue (18b) étant ainsi également déplacées l'une vers l'autre.

3. Dispositif selon l'une quelconque des revendications précédentes, dans lequel le dispositif (10) comprend en outre un élément de guidage (42), qui comprend une ouverture (44), qui est agencée perpendiculairement à l'axe médian longitudinal (28), et l'élément de guidage (42) s'applique sur les surfaces d'appui (40a, 40b) lorsque la deuxième force agit sur la section de retenue (16).

4. Dispositif selon l'une quelconque des revendications précédentes, dans lequel l'élément de couplage (20) comprend deux protubérances (46a, 46b), qui sont parallèles l'une à l'autre et entre lesquelles l'élément de poussée (22) est guidé.

5. Dispositif selon l'une quelconque des revendications précédentes, dans lequel l'élément de couplage (20) comprend deux évidements (48a, 48b), qui sont tournés l'un vers l'autre, et dans lequel la section de retenue (16) comprend sur deux côté opposés à chaque fois une protubérance (50a, 50b), qui pénètre dans un des évidements (48a, 48b), ou dans lequel l'élément de couplage (20) comprend deux protubérances, qui sont tournées l'une vers l'autre, et dans lequel la section de retenue (16) comprend sur deux côtés opposés à chaque fois un évidement, dans lequel une des protubérances pénètre.

6. Dispositif selon la revendication 5, dans lequel les évidements (48a, 48b) comprennent une fente (52a, 52b), qui est configurée de telle sorte que la section de retenue (16) peut être poussée hors des évidements (48a, 48b) perpendiculairement à l'axe de pivotement (24).

7. Dispositif selon l'une quelconque des revendications précédentes, dans lequel l'axe médian longitudinal (28) coupe l'axe de pivotement (24).

8. Dispositif selon l'une quelconque des revendications précédentes, en outre pourvu d'un bras de retenue (102), par lequel l'élément de poussée (22) est retenu lorsque la deuxième force est exercée, l'élément de couplage (20) comprenant une bride (54), qui est fixée au bras de retenue (102) avec un élément à chapeau (56).

9. Dispositif selon l'une quelconque des revendications précédentes, dans lequel l'élément de poussée (22) est couplée au bras de retenue (102) par l'intermédiaire d'une articulation sphérique (58).

10. Dispositif selon l'une quelconque des revendications précédentes, dans lequel un premier élément de retenue (18a) parmi les éléments de retenue (18a, 18b) comprend un retrait (60a) et un deuxième élément de retenue (18b) parmi les éléments de retenue (18a, 18b) comprend une protubérance (62a), la protubérance (62a) pénétrant dans le retrait (60a) lorsque la deuxième force agit sur la section de retenue (16).

11. Dispositif selon l'une quelconque des revendications précédentes, dans lequel chaque élément bras (32a, 32b) comprend une troisième section de bras (64a, 64b), qui est agencée entre la première section de bras (36a, 36b) et la deuxième section de bras (38a, 38b), et s'étend notamment au moins approximativement parallèlement à l'axe médian longitudinal (28).

12. Dispositif selon la revendication 11, dans lequel la première section de bras (36a, 36b) et la deuxième section de bras (38a, 38b) se situent à chaque fois au moins approximativement perpendiculairement à la troisième section de bras (64a, 64b).

13. Dispositif selon l'une quelconque des revendications précédentes, dans lequel un élément de retenue élastique est inséré dans la section de retenue (16), qui est configuré de manière à recevoir un premier instrument (12) parmi les instruments médicaux (12, 14) le long de l'axe médian longitudinal (28).

14. Dispositif selon l'une quelconque des revendications précédentes, dans lequel la section de retenue (16) est configurée pour guider un trocart, et les surfaces d'appui (40a, 40b) sont configurées pour retenir un endoscope introduit dans le trocart.

15. Système (104) pourvu d'un dispositif (10) selon l'une quelconque des revendications précédentes, d'un trocart et d'un endoscope, dans lequel le trocart est retenu par la section de retenue (16) le long de l'axe médian longitudinal (28) et l'endoscope est introduit dans le trocart et est retenu par une force exercée par les surfaces d'appui (40a, 40b).
